# FASCICULE DE BREVET EUROPEEN

(11) **EP 1 871 290 B1**
(45) Date de publication et mention de la délivrance du brevet: **15.09.2010**
(21) Numéro de dépôt: 06726061.2
(22) Date de dépôt: 09.03.2006
(51) Int. Cl.: A61F 2/06

(54) **DISPOSITIF DE MESURE DU DIAMETRE D'UN ANNEAU AORTIQUE**
VORRICHTUNG ZUR MESSUNG DES DURCHMESSERS EINES AORTENANNULUS
DEVICE FOR MEASURING THE DIAMETER OF AN AORTIC ANNULUS

(30) Priorité: 14.03.2005 FR 0502496
(43) Date de publication de la demande: 02.01.2008
(73) Titulaire: ASSISTANCE PUBLIQUE - HOPITAUX DE PARIS, 75001 Paris (FR)
(72) Inventeur: LANSAC, Emmanuel, F-75013 Paris (FR); KRAUSS, Christian, F-77240 Cesson (FR); DICENTA, Isabelle, F-75012 Paris (FR)
(74) Mandataire: Lagrange, Jacques Etienne M.M.
(86) Numéro de dépôt international: PCT/FR2006/000530
(87) Numéro de publication internationale: WO 2006/097604

(56) Documents cités:
- WO-A-96/40006
- US-A- 4 362 167
- US-A1- 2001 039 388
- US-B1- 6 322 526

## Description

La présente invention est relative à un dispositif de mesure du diamètre d'un anneau aortique et du diamètre interne de structures vasculaires et à son utilisation.

Lors de certaines opérations sur la racine aortique, telles que la mise en place d'un anneau aortique prothétique, il est nécessaire de mesurer le diamètre de l'anneau aortique natif.

Il est connu par les documents WO96/40006 et US 6 322 526 des moyens de mesure destinés à être utilisés dans le cadre d'une chirurgie de remplacement de la valve aortique avec excision de celle-ci.

Par ailleurs, actuellement, une telle mesure est effectuée sur un coeur arrêté dont la racine aortique n'est pas en charge, à l'aide d'un jeu de bougies de Hégar (cylindre de calibrage de 2 mm en 2 mm). Cet instrument est introduit par l'aortotomie et descendu dans la chambre de chasse du ventricule gauche. L'utilisation de ces bougies de Hégar donne un renseignement subjectif relatif au diamètre de l'anneau aortique du type «entre-entrepas ». En fonction du calibre de la bougie, on obtient l'information « bon » ou « mauvais » diamètre. Cependant, et compte tenu de la nature extensible de l'anneau aortique, une bougie ayant un calibre de 2 à 4 mm supérieur à celui de l'anneau aortique mesuré, peut être introduite sans difficultés dans la racine aortique. Il en résulte un calibrage assez approximatif de l'anneau aortique.

Le but de la présente invention est de remédier à cet inconvénient en proposant un moyen pour mesurer le diamètre interne de l'anneau aortique natif sur un coeur arrêté, qui soit bien représentatif du diamètre de l'anneau aortique natif d'une racine aortique sur un coeur en charge.

A cet effet, l'invention a pour objet un dispositif de mesure d'un anneau aortique comprenant, à son extrémité distale, une tête de mesure cylindrique expansible adaptée pour être introduite par l'aortotomie dans la chambre de chasse d'un ventricule gauche, à son extrémité proximale, un moyen de commande et de mesure à force de mesure constante et réglable de l'expansion de la tête, et une tige de liaison entre la tête de mesure et le moyen de commande et de liaison.

L'extrémité de la tête de mesure est non contondante et la tête de mesure a une longueur de préférence supérieure à 10 cm pour être adaptée à la hauteur de la chambre de chasse du ventricule gauche et de la racine aortique.

La longueur de la tige de liaison entre la tête de mesure et le mécanisme de commande et de mesure est de préférence supérieure à 8 cm pour permettre la manipulation du dispositif de mesure par le chirurgien à partir de l'extérieur du thorax.

De préférence, le dispositif de mesure est adapté pour mesurer un diamètre compris au moins entre 18 mm et 32 mm, avec une force de mesure comprise au moins entre 3 N et 10 N.

Le moyen de commande et de mesure de l'expansion de la tête est par exemple une vis micrométrique avec limitateur de couple.

La tête de mesure peut comprendre trois palettes allongées parallèlement à l'axe longitudinal du dispositif, et mobiles radialement par rapport à l'axe longitudinal du dispositif entre une position rétractée et une position expansée, ou comprendre un ruban expansible radialement par rapport à l'axe longitudinal du dispositif.

De préférence, le dispositif de mesures est constitué de matériaux adaptés à un usage en tant qu'instrument chirurgical.

L'invention est destinée à être utilisée notamment dans un procédé de mesure d'un anneau aortique natif sur un coeur arrêté, sous circulation extracorporelle et accessible par une aortotomie, selon lequel, par l'aortotomie, on introduit dans la chambre de chasse du ventricule gauche un dispositif de mesure selon l'invention dont la tête de mesure est rétractée et, à l'aide du moyen de commande et de mesure, on expanse la tête de mesure jusqu'à ce que la force de mesure limite soit atteinte, on repère alors le diamètre indiqué par le moyen de commande et de mesure et, après avoir rétracté les pallettes de la tête de mesure, on retire le dispositif de mesure.

L'invention va maintenant être décrite de façon plus précise mais non limitative en regard des figures annexées dans lesquelles :
- les figures 1A et 1 B représentent schématiquement un premier mode de réalisation d'un dispositif de mesure d'un anneau aortique natif, disposé dans ledit anneau, d'une part dans une position rétractée, d'autre part dans une position expansée ; et
- la figure 2 est une représentation schématique d'un deuxième mode de réalisation d'un dispositif de mesure d'un anneau aortique natif, disposé dans un anneau aortique, d'une part dans une position rétractée, d'autre part dans une position expansée.

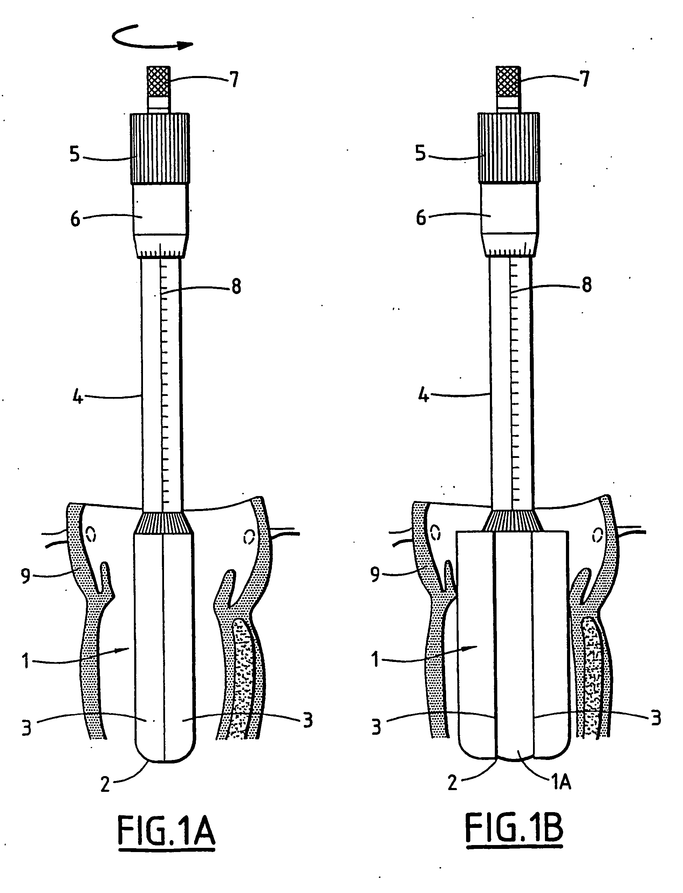

Dans un premier mode de réalisation représenté aux figures 1A et 1B, le dispositif de mesure de l'anneau aortique natif comprend une tête de mesure repérée généralement par 1, de forme cylindrique allongée, ayant une extrémité 2 arrondie et douce de façon à être non contondante. Cette tête de mesure est constituée de trois palettes 3 (on n'en voit que deux sur la figure 1A), qui s'étendent longitudinalement par rapport à l'axe longitudinal du dispositif, et qui sont mobiles radialement entre une position rétractée représentée à la figure 1A, et une position expansée représentée à la figure 1 B.

Les palettes sont montées sur un mécanisme 1A (non visible en détail sur la figure), connu de l'homme du métier, qui permet, en agissant sur un moyen de commande, de déplacer radialement les palettes tout en mesurant le déplacement de ces palettes. En particulier, le mécanisme peut être identique aux mécanismes connus en eux-mêmes de dispositifs de mesure du diamètre d'un alésage, utilisés en mécanique. La tête de mesure, dans sa position rétractée, a un diamètre sensiblement inférieur ou égal à 19 mm et mieux à 18 mm. Dans la position expansée, la tête de mesure à un diamètre supérieur ou égal à 31 et mieux à 32 mm.

La longueur de la tête de mesure est adaptée pour être compatible avec une bonne mesure du diamètre d'un anneau aortique natif. Cette longueur qui est supérieure à 10 cm est de préférence d'environ 15 cm,

La tête de mesure, repérée généralement par 1, est portée par une tige 4 à l'extrémité de laquelle est disposé un dispositif 5 de commande de la tête de mesure, de mesure et d'affichage de la mesure du diamètre de la tête de mesure. Ainsi, le dispositif de mesure comporte à son extrémité distale une tête de mesure et à son extrémité proximale un dispositif de commande et de mesure ou d'affichage de la mesure.

La tige 4 est d'une longueur adaptée pour pouvoir introduire le dispositif de mesure à l'intérieur du thorax du patient. Cette longueur est d'au moins 10 cm.

La tige 4 peut être creuse et contenir un axe (non visible sur la figure) permettant d'entraîner le mécanisme 1A d'expansion de la tête de mesure 1 à l'aide du dispositif de commande 5.

Le dispositif de commande, de mesure et d'affichage 5 est un dispositif du type vis micrométrique à limitateur de couple, connu en lui-même comportant un tambour 6, et un bouton 7. Le mécanisme limitateur de couple est connu en lui-même, et peut être du type bouton à cliquet, tambour mobile à cliquet ou tambour mobile à friction. Il permet d'effectuer une mesure à effort constant, c'est-à-dire une mesure pour laquelle la force maximale exercée par les palettes sur les parois de l'anneau aortique est constante et limitée. Cette force de mesure peut être réglée par les dispositifs connus en eux-mêmes de l'homme du métier, qui viennent d'être indiqués. Mais, de préférence, ils sont réglés à une valeur comprise entre 3 N et 10 N et de préférence entre 3,5 N et 9 N, pour permettre de réaliser des mesures de l'anneau aortique correspondant à la pression diastolique moyenne de la racine aortique d'un coeur en charge, environ 80 mm de mercure.

Le dispositif 5, associé à la tige 4, comporte des graduations 8 servant à afficher la mesure.

Cet appareil permet ainsi de mesurer le diamètre d'un anneau aortique natif 9 dans des conditions proches des conditions physiologiques normales, avec une précision de l'ordre de 1 mm.

Cet appareil permet ainsi de mesurer le diamètre d'un anneau aortique natif 9 dans des conditions proches des conditions physiologiques normales, avec une précision de l'ordre de 1 mm.

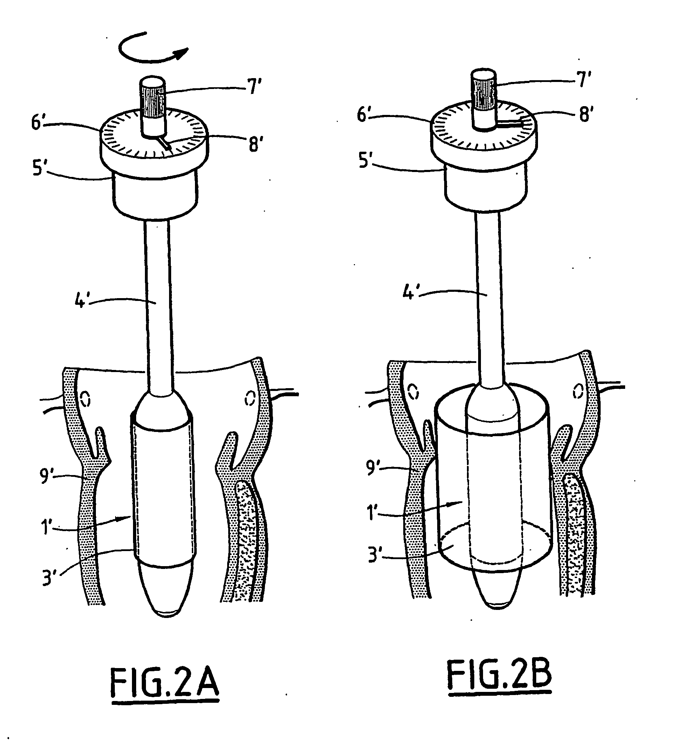

Dans un deuxième mode de réalisation repéré aux figures 2A et 2B, dans lesquelles on a utilisé les mêmes repères que dans les figures précédentes mais en y introduisant des primes, la tête de mesure 1', qui a une forme et une taille comparables à celles de la tête de mesure du cas précédent, tant pour ce qui est de la longueur que du diamètre, comporte une extrémité non contondante 2' et à sa périphérie un moyen de mesure constitué d'un ruban expansible 3'. Ce ruban est expansible radialement à l'aide d'un mécanisme connu de l'homme du métier.

La tête de mesure, adaptée pour être introduite dans un anneau aortique natif 9', est située à l'extrémité distale de l'appareil et portée par une tige 4' dont la longueur, comme dans le cas précédent, est d'au moins 10 cm, et à l'extrémité de laquelle est disposé un moyen 5' de commande de la tête de mesure et d'affichage de la mesure.

Ce moyen de commande et d'affichage se situe à l'extrémité proximale du dispositif.

Dans le mode de réalisation représenté à la figure 2A ou 2B, le moyen de commande et d'affichage 5' comporte un disque gradué 6', et un piston de commande 7' destiné à faire varier le diamètre du ruban de mesure 3', et qui comporte une aiguille 8' indiquant le diamètre défini par le ruban de mesure 3'. Le dispositif comporte en outre un limitateur d'effort, par exemple par friction, pour que, lorsque le ruban 3' bute sur les parois d'un anneau aortique natif 9' et que la force atteint une valeur définie par avance, le mouvement du ruban 3' soit stoppé même si l'opérateur continue à exercer un effort sur le piston 7'.

Avec ce dispositif, la mesure se fait dans les mêmes conditions qu'avec le dispositif décrit précédemment.

Plus précisément lorsqu'un chirurgien souhaite mesurer le diamètre d'un anneau aortique natif, il introduit le dispositif de mesure qui vient d'être décrit, à l'état rétracté, par l'ouverture du thorax et l'aortomie, dans la chambre de chasse du ventricule gauche d'un coeur arrêté sous circulation extra corporelle.

Lorsque la tête de mesure est bien positionnée à l'intérieur de l'anneau aortique natif, à l'aide du dispositif de commande et d'affichage, le chirurgien génère l'expansion de la tête de mesure jusqu'à ce qu'elle vienne buter sur l'anneau aortique natif et qu'elle soit bloquée parce que la force de mesure est atteinte. Lorsque la force de mesure est atteinte, le chirurgien lit sur les moyens d'affichage le diamètre de l'anneau aortique natif. Il peut alors rétracter le dispositif de mesure et le ressortir du corps du patient.

Le fait d'effectuer la mesure avec une force de mesure constante correspondant à une pression aortique diastolique moyenne a pour avantage de conduire à une mesure du diamètre proche des conditions physiologiques normales lorsque la valve aortique est fermée et que la racine aortique est en charge.

On notera que, dans tous les cas, la tête de mesure qui est allongée, a un contact linéaire avec la paroi interne de l'anneau aortique, ce qui assure un auto-alignement de l'instrument perpendiculairement à la section de l'anneau aortique.

Le dispositif, destiné à être utilisé essentiellement dans des conditions chirurgicales, est constitué de matériaux compatibles avec son utilisation en tant qu'instrument chirurgical, c'est-à-dire, des matériaux bien adaptés à la stérilisation et biocompatibles. Ce sont par exemple des aciers inoxydables adaptés à la fabrication d'instruments chirurgicaux.

Les deux modes de réalisation qui viennent d'être décrits ne sont pas limitatifs et tout dispositif qui utilise une tête de mesure expansible radialement de forme adaptée à l'utilisation dans un anneau aortique, et comportant des moyens pour exercer un effort de mesure limité et constant, peut être utilisé pour la réalisation d'un dispositif de mesure selon l'invention.

## Revendications

1. Dispositif de mesure du diamètre d'un anneau aortique comprenant, à son extrémité distale, une tête de mesure (1, 1') cylindrique expansible adaptée pour être introduite par l'aortotomie dans la chambre de chasse d'un ventricule gauche, à son extrémité proximale, un moyen (5, 5') de commande et de mesure de l'expansion de la tête, et une tige de liaison (4, 4') entre la tête de mesure et le moyen de commande et de mesure, **caractérisé en ce que** le moyen de commande et de mesure est à force de mesure constante et réglable, c'est-à-dire que la force maximale exercée par la tête de mesure sur les parois de l'anneau aortique est réglable à une valeur constante et limitée.

2. Dispositif selon la revendication 1, **caractérisé en ce que** l'extrémité (2, 2') de la tête de mesure (5, 5') est non contondante.

3. Dispositif selon la revendication 1 ou la revendication 2, **caractérisé en ce que** la tête de mesure (1, 1') a une longueur supérieure à 10 cm, adaptée à la mesure d'un anneau aortique.

4. Dispositif selon l'une quelconque des revendications 1 à 3, **caractérisé en ce que** la longueur de la tige de liaison (4, 4') entre la tête de mesure (1, 1') et le mécanisme de commande et de mesure (5, 5') est supérieure à 8 cm et adaptée pour permettre une mesure à partir de l'extérieur du thorax d'un patient.

5. Dispositif selon l'une quelconque des revendications 1 à 4, **caractérisé en ce qu'**il est adapté pour mesurer un diamètre compris au moins entre 18 mm et 32 mm, avec une force de mesure comprise au moins entre 3 N et 10 N.

6. Dispositif selon l'une quelconque des revendications 1 à 5, **caractérisé en ce que** le moyen (5) de commande et de mesure de l'expansion de la tête est une vis micrométrique avec limitateur de couple.

7. Dispositif selon l'une quelconque des revendications 1 à 6, **caractérisé en ce que** la tête de mesure (1) comprend trois palettes (3) allongées parallèlement à l'axe longitudinal du dispositif et mobiles radialement par rapport à l'axe longitudinal du dispositif.

8. Dispositif selon l'une quelconque des revendications 1 à 6, **caractérisé en ce que** la tête de mesure (1') comprend un ruban (3') expansible radialement par rapport à l'axe longitudinal du dispositif.

9. Dispositif selon l'une quelconque des revendications 1 à 8, **caractérisé en ce qu'**il est constitué de matériaux adaptés à un usage en tant qu'instrument chirurgical.

## Claims

1. Device for measuring the diameter of an aortic annulus which comprises, at its distal end, a cylindrical expansible measuring head (1, 1') which is adapted in order to be introduced by aortotomy into the left ventricular outflow tract, at its proximal end, a means (5, 5') for controlling and for measuring the expansion of the head, and a connection rod (4, 4') between the measuring head and the control and measuring means, **characterised in that** the control and measuring means is by force of a constant and regulatable size, i.e. the maximum force exerted by the measuring head on the walls of the aortic annulus can be regulated to a constant and limited value.

2. Device according to claim 1, **characterised in that** the end (2, 2') of the measuring head (5, 5') is not blunt.

3. Device according to claim 1 or claim 2, **characterised in that** the measuring head (1, 1') has a length which is greater than 10 cm, adapted to the size of an aortic annulus.

4. Device according to any of the claims 1 to 3, **characterised in that** the length of the connection rod (4, 4') between the measuring head (1, 1') and the control and measuring mechanism (5, 5') is greater than 8 cm and adapted in order to allow measurement from the exterior of the thorax of a patient.

5. Device according to any of the claims 1 to 4, **characterised in that** it is adapted in order to measure a diameter at least between 18 mm and 32 mm, and with a measuring force at least between 3 N and 10 N.

6. Device according to any of the claims 1 to 5, **characterised in that** the means (5) for controlling and for measuring the expansion of the head is a micrometer screw with a torque limiter.

7. Device according to any of the claims 1 to 6, **characterised in that** the measuring head (1) comprises three blades (3) which extend parallel to the longitudinal axis of the device and are radially moveable relative to the longitudinal axis of the device.

8. Device according to any of the claims 1 to 6, **characterised in that** the measuring head (1') includes a band (3') which is expansible radially relative to the longitudinal axis of the device.

9. Device according to any of the claims 1 to 8, **characterised in that** it consists of materials which are adapted for use as a surgical instrument.

## Patentansprüche

1. Vorrichtung zum Messen des Durchmessers eines Aortenringes, umfassend einen zylindrischen, expandierbaren Messkopf (1, 1') an ihrem distalen Ende, dazu geeignet, bei der Aortotomie in den Aortenkanal der linken Herzkammer eingeführt zu werden, ein Mittel (5, 5') zur Steuerung und Messung der Expandierung des Kopfes, an ihrem proximalen Ende, und einen Verbindungsstab (4, 4') zwischen dem Messkopf und dem Mittel zur Steuerung und Messung, **dadurch gekennzeichnet, dass** das Mittel zur Steuerung und Messung eine konstante, einstellbare Messkraft aufweist, das heißt, dass die vom Messkopf auf die Wände des Aortenringes ausgeübte maximale Kraft auf einen konstanten und begrenzten Wert einstellbar ist.

2. Vorrichtung gemäß Anspruch 1, **dadurch gekennzeichnet, dass** das Ende (2, 2') des Messkopfes (5, 5') nicht stumpf ist.

3. Vorrichtung gemäß Anspruch 1 oder Anspruch 2, **dadurch gekennzeichnet, dass** der Messkopf (1, 1') eine Länge von mehr als 10 cm hat, die zum Messen eines Aortenringes geeignet ist.

4. Vorrichtung gemäß einem der Ansprüche 1 bis 3, **dadurch gekennzeichnet, dass** die Länge des Verbindungsstabs (4, 4') zwischen dem Messkopf (1, 1') und dem Steuer- und Messmechanismus (5, 5') mehr als 8 cm beträgt und geeignet ist, eine Messung von außerhalb des Thorax eines Patienten zu gestatten.

5. Vorrichtung gemäß einem der Ansprüche 1 bis 4, **dadurch gekennzeichnet, dass** sie geeignet ist, einen Durchmesser zu bestimmen, der mindestens zwischen 18 mm und 32 mm liegt, bei einer Messkraft, die mindestens zwischen 3 N und 10 N liegt.

6. Vorrichtung gemäß einem der Ansprüche 1 bis 5, **dadurch gekennzeichnet, dass** das Mittel (5) zur Steuerung und Messung der Expandierung des Kopfes eine Mikrometerschraube mit Drehmomentbegrenzer ist.

7. Vorrichtung gemäß einem der Ansprüche 1 bis 6, **dadurch gekennzeichnet, dass** der Messkopf (1) drei parallel zur Längsachse der Vorrichtung ausgerichtete und bezogen auf die Längsachse der Vorrichtung radial bewegliche, längliche Paletten (3) umfasst.

8. Vorrichtung gemäß einem der Ansprüche 1 bis 6, **dadurch gekennzeichnet, dass** der Messkopf (1') ein bezogen auf die Längsachse der Vorrichtung radial dehnbares Band (3') umfasst.

9. Vorrichtung gemäß einem der Ansprüche 1 bis 8, **dadurch gekennzeichnet, dass** sie aus Werkstoffen gebildet ist, die für eine Nutzung als chirurgisches Instrument geeignet sind.
